# EUROPEAN PATENT APPLICATION

(11) **EP 4 728 995 A1**
(43) Date of publication of application: **22.04.2026**
(21) Application number: 24383153.4
(22) Date of filing: 21.10.2024
(51) Int. Cl.: A61B 10/00, A61B 10/02

(54) **A SALIVA COLLECTION DEVICE FOR COLLECTING A SALIVA SAMPLE OF A USER AND CORRESPONDING SALIVA EXTRACTION DEVICE**

(71) Applicant: Happy Innova SL, 17003 Girona (ES)
(72) Inventor: Valeta Arcarons, Albert, 08037 Barcelona (ES); Ballart Cerdán, Diana, 17490 Llançà (ES); García Puig, Roger, 08221 Terrassa (ES)
(74) Representative: Curell Suñol S.L.P.

(57) **Abstract**

A saliva collection device (1) for collecting a saliva sample of a user comprising handhold means (2) and an absorbent saliva collection pad (8) secured to a first end (4) to extend in a longitudinal direction (L). The collection device further comprises a pad protecting cover (10), releasably secured to the handhold means (2) in a pad covering position, in which the pad (8) is protected from deformation during a saliva sample collection procedure, and being movable relative to the handhold means (2), out of the pad covering position, to a pad releasing position, to extract the saliva sample. The protecting cover (10) further comprises a plurality of first inlet through holes (12) to provide a fluidical connection during the saliva sample collection procedure. The invention further relates to a saliva extraction device (100) for extracting a saliva sample from a saliva collection device (1) according to the invention.

## Description

### Field of the invention

The invention relates to a saliva collection device for collecting a saliva sample of a user comprising handhold means with a first and a second ends, said handhold means extending in a longitudinal direction, and an absorbent saliva collection pad, said collection pad being secured to said first end of said handhold means to extend in said longitudinal direction.

The invention further relates to a saliva extraction device for extracting a saliva sample, comprising a saliva collection device according to the invention.

### State of the art

Saliva collection devices are known in the art both for medical as well as for veterinary purposes. These devices are intended to collect saliva samples for health diagnostics or the detection of substances such as drugs, alcohol, or similar analytes. Numerous studies have validated the reliability and accuracy of saliva-based analyses compared to blood samples, particularly for detecting antibodies, hormones, and genetic material. Despite this, most saliva collection technologies have been developed with an "adultcentric" approach, relying on the subject's voluntary and active participation, which poses challenges when dealing with populations that may not be able to cooperate fully. Research has shown that participants often prefer saliva collection due to its simplicity and reduced discomfort, leading to higher compliance rates in longitudinal studies. As saliva has been firmly established as a valuable diagnostic medium, there remains an unmet need for devices that enable easy and effective collection, especially in paediatric populations or in adults with neuropsychiatric conditions, disabilities, or needle phobia, who may struggle to comply with standard collection methods. Developing non-invasive, user-friendly devices for these groups would significantly expand the applicability and accessibility of saliva-based diagnostics

Document US 2006/074347 A1 discloses an easy-to-gasp sputum collecting tool facilitating sputum collection work. The sputum-collecting tool has a sputum-collecting device with a base part having a sputum-collecting portion for collecting sputum at one end and a handle portion at the other end, and a collection sample container for receiving the sputum-collecting device inside it. This device has the problem that the sputumcollection portion hinders the collection of large amounts of sputum from the mouth of the user.

Document EP 3007824 A1 discloses an apparatus to obtain RNA-enhanced and proteinenhanced fluid samples which are stable at ambient temperatures. The device can be used to collect fluid, and to then extract and prepare the fluid for analysis. The device has also a cylindric collection pad which is not comfortable to the user for the oral fluid collection. That can eventually reduce the saliva production of the user.

Document US 11419587 B2 discloses a device for collecting fluid, and to then extract and prepare the fluid for analysis, which can eliminate human error in collection and reduce complexity in diagnostic analysis. The disclosed device may be used to collect any liquid specimen. Any instance when liquid should be collected, stored, and tested may benefit from use of this disclosure. Again, the pad is uncomfortable for the user and the amount of collected saliva is not as large as desirable.

Furthermore, the devices of the prior art described above are conceived for adult users, that is users that consciously collaborate in the saliva collection process. In other words, in the case of paediatric patients, patients with psychomotor difficulties of the oral cavity or animals this kind of devices is not so adequate to collect large amounts of saliva.

### Summary of the invention

It is an object of the invention to propose a saliva collection device for collecting a saliva sample of a user in which larger amounts of saliva can be collected, but also extracted afterwards easily, once the sample has been collected. This purpose is achieved by a saliva collecting device of the type indicated at the beginning, characterized in that it further comprises a pad protecting cover, said protecting cover being releasably secured to said handhold means in a pad covering position, in which said pad is protected from deformation during a saliva sample collection procedure, and being movable relative to said handhold means, out of said pad covering position, to a pad releasing position, to release said collection pad after said saliva sample collection procedure has been finished, to extract said saliva sample, and said protecting cover further comprising a plurality of first inlet through holes, said plurality of first inlet through holes being open towards said collection pad to provide a fluidical connection with said collection pad when said pad protecting cover is in said pad covering position during said saliva sample collection procedure.

The solution according to the invention has many synergistic advantages over the prior art device.

First, one of the relevant problems identified in the prior art devices is that the saliva collection pad is unprotected. Due to this, the user can bite of the pad during the saliva collection or simply squeeze the pad against the surfaces of his mouth cavity. In other words, every time that the user bites or squeezes the pad, the collected sample is at least partly released from the pad, making the collection very inefficient. Instead in the device of the invention, the saliva can enter the device through the inlet holes of the pad protecting cover towards the collection pad. However, once the pad is wet it remains protected by the cover without possible deformation. Therefore, even if the user bites the cover, the pad cannot be squeezed or compressed thus causing the drainage of the sample contained in the pad. Thus, the saliva can be collected very efficiently.

Secondly, the other advantage relates to the possibility of withdrawing the pad protecting cover once the cover is released from its secured situation, into the pad releasing position to release the pad after the sample collection and extract the saliva sample. By doing this, the effect reached is precisely the opposite to what was intended during the sample collection. Now the pad is released and fully compressible to drain the saliva sample in any adequate container or sample containing device. Again, this provides for a maximization in the saliva sample extraction.

Finally, the possibility of moving the pad protecting cover into the pad releasing position does not represent a danger for the user. In case the user removed the pad protecting cover during the saliva collection process, e.g. by biting and pulling the cover, the first inlet holes avoid the risk of airway obstruction of the user.

The invention further includes a number of preferred features that are object of the dependent claims and the utility of which will be highlighted hereinafter in the detailed description of an embodiment of the invention.

In a preferred embodiment, said protection cover is a sleeve open at one end remote from said handhold means, such as to allow the withdrawal of said sleeve with a compression force on said remote end, to release said collection pad through said remote end. This solution allows for the withdrawal of the cover with a simple compression effort on the free edge of the cover. Then the cover can be withdrawn without the need of touching it with the hand which is especially adequate to avoid any risk of cross contamination of the sample due to inadequate manipulation of the device during the withdrawal.

In another embodiment seeking for an increased collection amount of saliva, the saliva collection device further comprises a salivation enhancing layer, said salivation enhancing layer being made of a saliva properties-preserving composition and covering at least partially said cover in the longitudinal direction and comprising a plurality of second inlet through holes, said first and second inlet through holes matching each other at least partially. This layer is made of a saliva properties-preserving composition, both for the user and for the measurements.

Furthermore, the salivation enhancing layer can have a very reduced thickness or have less than 2 mm of thickness. In any case, it is intended that the salivation enhancing layer is fully removed when the saliva collection process has finished.

In the context of the invention, a saliva properties-preserving composition is to be understood as a composition specifically designed to preserve the chemical properties of the saliva sample, thereby enabling accurate laboratory analysis of analytes.

Therefore, the only function of the saliva properties-preserving composition is to stimulate the salivary glands to produce more saliva. This layer is preferably soluble with saliva. Also preferred tastes for the salivation enhancing layer are flavours such as orange, lemon, strawberry or the like, making for the user a more pleasant experience to obtain the saliva sample, and again facilitating the saliva secretion.

Especially preferably, said salivation enhancing layer is free of citric acid to avoid pH alterations in the sample that could cause modifications in the result of the later analysis.

Preferably, to reach a good securing of the salivation enhancing layer, said salivation enhancing layer is attached to the pad protection cover by means of a food grade adhesive. Alternatively, said salivation enhancing layer is attached to the pad protection cover by means of a thermal treatment causing an adhesion of said salivation enhancing layer to said pad protection cover.

Also preferably, said pad protection cover surface has a rough finishing for promoting the adhesion of the salivation enhancing layer.

Preferably, to provide an improved user experience leading also to a larger amount of collected saliva, in the device of the invention said salivation enhancing layer is a candy layer. Again, this allows that the user holds the device longer in his mouth, thus facilitating more saliva to be collected. Furthermore, this embodiment is especially adequate for paediatric patients. For children it can be disturbing and stressing to make this kind of collection procedures. A device configured as a lollipop makes the experience less disturbing for the children and helps to quite the ambience, making the child willing to collaborate in a more positive attitude. Again, this assures for larger amounts of saliva to be collected and facilitates the later tests.

In an especially preferred embodiment, the collection device comprises first retaining means arranged between said pad protecting cover and said handhold means, for releasably securing said pad protecting cover and said handhold means relative to each other in said pad covering position.

Another one of the objects of the invention is to improve the security of use of the device. To this end, preferably said first retaining means comprises at least one retaining hole and at least matching first retaining protrusion functionally arranged in said pad protecting cover and said handhold means, such that in said pad covering position, said first retaining protrusion is inserted into said at least one retaining hole. This positive fit union provides for a more secure connection between both parts, making almost impossible that the cover can be withdrawn simply by pulling on the cover with the user's teeth during the saliva collection process. However, in the unlikely event that this happened, still the first inlet holes provide for the airway's protection of the user if the user swallowed the pad protecting cover.

In an especially preferred embodiment, said protrusion has an insertion chamfer on the side facing the first end and a retaining shoulder on the side facing the second end. The chamfer allows the cover to be withdrawn only by compressing the cover against the handhold means. Instead, a pulling effort in the opposite direction causes the shoulder to retain the cover in the pad covering position, avoiding that the cover can be swallowed accidentally by the user, should he pull the cover away from the handhold means, e.g. by pulling the cover with the teeth. Therefore, this provides an increased security of use.

In an alternative embodiment seeking for a more sealing retention, said first retaining means comprises matching threads between said pad protecting cover and said handhold means, for releasably securing said pad protecting cover and said handhold means relative to each other in said pad covering position.

Also, to reach an uniform squeezing of the saliva collection pad and avoiding any damage on it during the releasing process of the cover, the collection device of the invention comprises first longitudinal guiding means arranged between said pad protecting cover and said handhold means, for guiding a longitudinal sliding movement of said pad protecting cover and said handhold means relative to each other, when said pad protecting cover is moved from said pad covering position into said pad releasing position during which said pad protecting cover and said handhold means can freely slide relative to each other in a longitudinal sliding movement in said longitudinal direction from said first end towards said second end.

Preferably, said first longitudinal guiding means comprises at least one guiding recess and a guiding protrusion and in that said guiding recess and said guiding protrusion cooperate mutually for guiding a longitudinal sliding movement. Thanks to the withdrawal of the cover is easier.

Another object of the invention to propose a saliva extraction device for extracting a saliva sample from a saliva collection device that facilitates the extraction, reduces the risk of cross contamination and leakage of the sample and maximizes the saliva extraction from the saliva collected before with the saliva collection device.

To solve this problem, the invention proposes a saliva extraction device for extracting a saliva sample from a saliva collection device according to the invention characterized in that said saliva extraction device further comprises a saliva extraction cylinder extending in a longitudinal direction and comprising a third and a fourth ends and a compression wall between said third and fourth ends, said wall comprising a saliva outlet nozzle protruding from said compression wall towards said fourth end and cover withdrawing means arranged to the side of said wall opposite to said saliva outlet nozzle for withdrawing said cover of said saliva collection device, a saliva sample container fluidly connected to said saliva outlet nozzle for receiving said saliva sample, and securing means between said saliva extraction cylinder and said saliva sample container to removably secure said saliva sample container to said saliva extraction cylinder, such as to receive said saliva sample flowing from said saliva outlet nozzle, said extraction cylinder being further configured for receiving said saliva collection device through said third end in a movable manner between a remote position in which said pad is uncompressed, and a final extraction position in which said pad protecting cover is withdrawn with a free edge of said pad protecting cover abutting against said cover withdrawing means and said pad is fully compressed against said compression wall for extracting a saliva sample contained in said pad through said saliva outlet nozzle into said saliva sample container.

Thanks to the extraction device, the saliva collected with the collection device of the invention can be easily extracted without the risk of cross contamination due to the contact with the hands of the collection pad, e.g. with the user tries to withdraw the cover with the hands. Furthermore, the sample is collected directly into the saliva sample container without any possible contact producing contamination or leakage.

Preferably, to reach a more ergonomic use of the saliva extraction device, said saliva extraction cylinder is at least 8 cm long for it to be held with one closed hand of the user. This minimum size allows the device to be used and firmly manipulated with one single hand during the extraction process.

Also preferably, said saliva extraction cylinder inner face tapers in the direction from said third end towards said compression wall. In this way a smooth insertion of the saliva collection device is guaranteed. Preferably, said saliva extraction cylinder inner face comprises at least three longitudinal insertion rib tapering in the direction from said third end towards said compression wall. This reduces the insertion friction and provides a more ergonomical extraction process.

In preferred embodiment, said securing means comprises at least three first longitudinal ribs formed in said saliva extraction cylinder to provide a press fit securing of said saliva sample container onto said saliva extraction cylinder. This configuration provides a reliable securing of the saliva sample container simply by pressing the saliva sample container against the first longitudinal ribs.

In an alternative embodiment, said securing means comprises matching threads between said extraction cylinder and said saliva sample container to provide a threaded connection between both parts. Although this configuration provides a more solid securing, it requires more manipulation by the user during the coupling and removing procedures.

In yet another embodiment, said securing means is a luer lock fitting between said extraction cylinder and said saliva sample container, to achieve a fast locking turning assembly.

Preferably, said at least three ribs protrude from the inner surface of the of the outer walls of said saliva extraction cylinder, to reduce the risk of contact with the saliva sample and the extraction cylinder. However, in an alternative embodiment for larger saliva sample containers, said at least three ribs can protrude from the outer surface of the saliva outlet nozzle.

During the development of the invention, it has been learned that the presence of bubbles in the sample either coming from the own sample, or created during the extraction, lead to the extraction of a smaller sample amount. Additionally, an excessive amount of bubbles can lead to analysing errors in the sample analysis machines and to misleading results. Therefore, to solve this problem, between said saliva outlet nozzle and said saliva sample container at least one air outlet is formed. It has been ascertained that providing an air outlet nozzle precisely in this extraction area and not far removed from the saliva extraction point, facilitates that the air of the saliva sample is removed, but also, synergistically, it is avoided that bubbles are formed. This has the synergistic effect that the saliva flows easier and thus a larger quantity of saliva is collected into the saliva sample container.

Another problem to solve, is to avoid that during the extraction of bubbles of the sample, part of the sample exits through the air outlet. To this end, preferably, said compression wall comprises at least a first abutment protrusion on the side of said outlet nozzle and when said saliva sample container is removably secured to said saliva extraction cylinder a front edge of said saliva sample container abuts against said first abutment protrusion. With this solution, a labyrinth structure is created that avoids that the saliva can overflow the sample container when it drips through the compression wall and the outlet nozzle towards the saliva sample container.

Preferably, said at least one abutment protrusion is a radial abutment rib, and more preferably said protrusion are at least three ribs. Indeed, it is enough that a certain distance is kept relative to the compression wall, but at least three ribs provide for a better positioning of the saliva sample container.

Also, to increase the amount of extracted saliva from the pad, said compression wall comprises at least three extraction ribs on the side of said compression wall opposite to said outlet nozzle protruding from said compression wall, such that in said compressed position, in which said pad is compressed against said compression wall for extracting a saliva sample, said pad is compressed against said at least three extraction ribs. This configuration has the advantage of creating a draining chamber below the extraction ribs that facilitate the saliva to flow downwards towards the compression wall.

Also to accelerate the sample extraction procedure, on the side of said compression wall opposite to said outlet nozzle, said compression wall is shaped as a funnel, such that in use said collected saliva can flow into said saliva outlet nozzle. The funnel promotes the saliva circulation from the draining chamber. This accelerates the extraction but also increases the amount of saliva extracted.

Preferably said compression wall is shaped as funnel forming an angle comprised between 90 and 165°.

In another approach to increase the saliva extraction speed, said saliva outlet nozzle is shaped as a tubular duct extending in the longitudinal direction and in that the end of said tubular duct remote from said compression wall is finished in a plane diagonally oriented relative to said longitudinal direction. This shape forms a spout increasing again not only the extraction speed, but also the amount of saliva extracted in the saliva sample container. The spout helps breaking the surface tension of the saliva sample, making it flow better downwards towards the saliva sample container.

Finally, in order to help the user knowing when the collection pad is fully compressed for completely extracting sample, said handhold means comprises a flange and said saliva extraction cylinder comprises at least one final stroke protrusion on the inner surface of said saliva extraction cylinder, said flange and said final stroke protrusion cooperate mutually to produce a tactile feedback when said saliva collection device has reached said final extraction position. The user simply needs to compress the end of the saliva collection device until he feels a little click feeling coming from the interaction between the cylinder and the handhold means of the saliva collection device. At this point, the user knows that he needs only to hold this position for some seconds, to give time to the sample to flow downwards towards the sample container, without the risk that the pad is newly soaked with the saliva sample flowing backwards. Furthermore, this tactile feedback means also serves for retaining the saliva collection device in the saliva extraction device, thus reducing the biohazard risk and improving the hygiene of use of the assembly.

Especially preferably said pad protecting cover is so long that in said final extraction position in which said pad protecting cover is withdrawn to said pad releasing position, said cover simultaneously abuts against said flange and said cover withdrawing means. This provides also a reliable feeling to the user that the extraction is performed correctly.

Likewise, the invention also includes other features of detail illustrated in the detailed description of an embodiment of the invention and in the accompanying figures.

### Brief description of the drawings

Further advantages and features of the invention will become apparent from the following description, in which, without any limiting character, preferred embodiments of the invention are disclosed, with reference to the accompanying drawings in which:
Figure 1, a diagrammatical exploded view of the saliva extraction device according to the invention.
Figure 2, a longitudinal section view of the saliva collection device according to the invention with a salivation enhancing layer.
Figure 3, a longitudinal section view of the saliva collection device according to the invention without a salivation enhancing layer.
Figure 4, a detailed section view of the saliva collection device of Figure 3.
Figure 5, a longitudinal section view of the saliva collection device of Figure 3, during the withdrawal procedure of the pad collection cover.
Figure 6, a detailed section view of the saliva collection device of Figure 3.
Figure 7, an isometric longitudinal section view of the saliva collection device of Figure 3.
Figure 8, a cross-section view of the handhold means and the pad protecting cover of the saliva collection device of Figure 3.
Figures 9 to 11, longitudinal section views of a saliva extraction device according to the invention with a saliva collection device inserted in the saliva extraction device during the extraction procedure.
Figure 12, a longitudinal section view of the cylinder of the saliva extraction device of figures 9 to 11,
Figure 13, a detailed longitudinal section view of the cylinder of the saliva extraction device of figures 9 to 11, and
Figures 14 to 17, longitudinal section views in perspective of the cylinder of the saliva extraction device of figures 9 to 11.
Figure 18, a bottom view of the cylinder of the saliva extraction device of figures 9 to 11.
Figure 19, a longitudinal section view of a second embodiment of the saliva collection device according to the invention without a salivation enhancing layer.
Figure 20, a longitudinal section view of the cylinder of a second embodiment of a saliva extraction device according to the invention, without sample container,

### Detailed description of embodiments of the invention

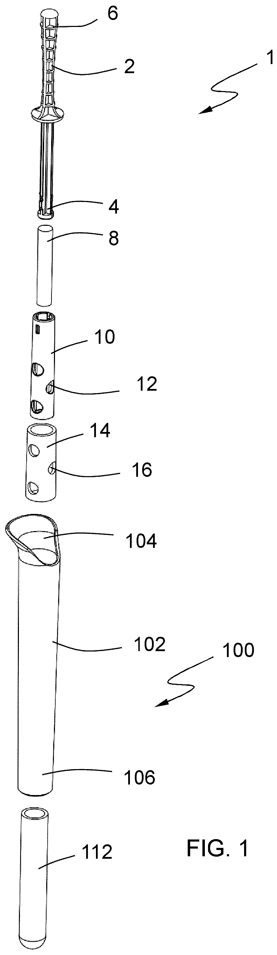

Figures 1 to 8 show a saliva collection device 1 for collecting a saliva sample of a user according to the invention.

The saliva collection device 1 is intended both for medical, as well as for veterinary use, both for adults, as well as paediatric applications.

As main components, the saliva collection device 1 comprises handhold means 2, a saliva collection pad 8 and a pad protecting cover 10.

The handhold means 2 have a first and a second ends 4, 6 and extending in a longitudinal direction L.

The absorbent saliva collection pad 8 is secured to the first end 4 of the handhold means 2 to extend in the longitudinal direction L. In this case, the saliva collection pad 2 has the shape of a circular cylinder and is made of a porous, foam or sponge like material, such as cellulose, synthetic swab, porous plastics, porous fibers or porous foam or the like. One example of material adequate for this application is the SalivaBio Children's Swab device of the company Salimetrics, LLC. The saliva collection pad 8 is compressible and hydrophilic to absorb the saliva from the user's mouth during the saliva collection procedure. The saliva collection pad 8 is secured to the first end 4 of the handhold means 2, e.g. by an adhesive layer, thermo-soldering or the like, but it is not shown in detail.

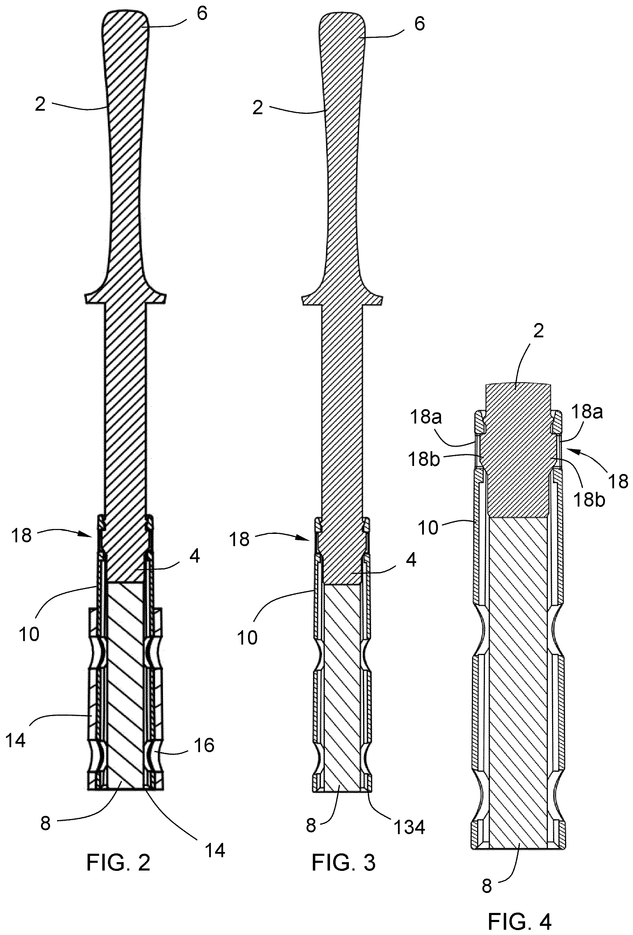

The pad protecting cover 10 is releasably secured to the handhold means 2 in a pad covering position. In this first position shown in Figures 2 or 3, the pad 8 is protected from deformation during a saliva sample collection procedure. As already mentioned, this avoids the accidental deformation and early draining of the pad 8 during the collection procedure, which would negatively impact the amount of saliva contained in the sample.

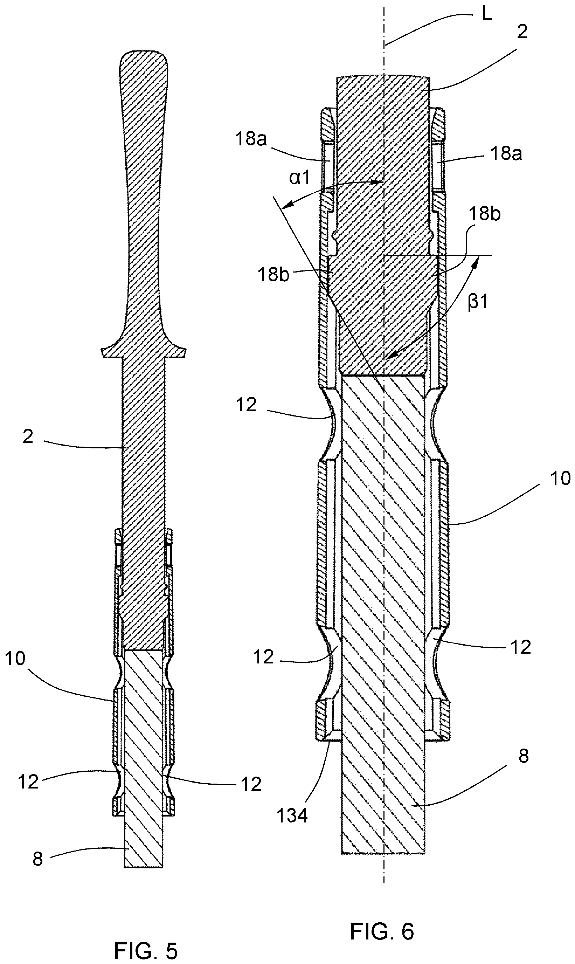

Furthermore, the pad protecting cover 10 of the saliva collection device 1 of the invention is movable relative to the handhold means 2, out of the pad covering position, to a pad releasing position, only if the securing force has been overcome. In this position, the collection pad 8 is released after the saliva sample collection procedure has been finished, to extract said saliva sample. In other words, the cover 10 is secured to handhold means 2 in a way that to release the cover 10 from the position of Figure 3, into the position of Figures 5 or 6, a compression force between 20 and 60 N has to be exerted. In the opposite pulling direction this force can set be even higher as explained below, for security purposes.

Preferably, to firmly secure the cover 10 to the handhold means 2, as shown in figures 2 to 4, the saliva collection device 1 comprises first retaining means 18 arranged between the pad protecting cover 10 and the handhold means 2. This retaining means 18 releasably retain the pad protecting cover 10 and the handhold means 2, relative to each other in the pad covering position. More particularly in this embodiment, the first retaining means 18 comprises two diametrically opposed retaining holes 18a (see Figure 4) and two matching first retaining protrusions 18b, being functionally arranged in the pad protecting cover 10 and the handhold means 2, such that in the pad 8 covering position, the first retaining protrusions 18b are inserted into the corresponding retaining holes 18a. In this case, the retaining holes 18a are provided in the cover 10, and the protrusions 18b are provided in the handhold means 2, but this could also work the other way around.

In this preferred embodiment, the protrusions 18b have an insertion chamfer on the side for the protrusions 18b facing the first end 4, while a retaining shoulder is arranged on the side of the protrusions 18b facing the second end 6 of the handhold means 2. Preferably, as shown in Figure 6, the insertion chamfer defines an angle α1 relative to the longitudinal direction L comprised between 10° and 45°. The chamfer, apart from aiding to the assembly process of the cover 10 on the handhold means 2 of the saliva collection device 1, has the additional function of allowing the cover 10 to be released from its secured pad covering position only by pushing cover 10 against the handhold means 2. Also, in Figure 6 can be seen that preferably, the retaining shoulder defines an angle β1 relative to the longitudinal direction L of at least 75° and preferably at least 90°. Instead, a pulling effort in the opposite direction causes the shoulders to retain the cover 10 in the pad 8 covering position, avoiding that the cover 10 is separated and later be swallowed accidentally by the user, should he pull the cover away from the handhold means, e.g. by pulling the cover with the teeth. In the invention a traction force comprised between 60 N and 130 N has been proved adequate for avoiding this kind of casualties and increasing the security of use of this preferred embodiment.

Other embodiments of the first retaining means 18 arranged between the pad protecting cover 10 and the handhold means 2 are conceivable in the invention.

Alternatively, this first retaining means 18 can be a threaded union between the pad protecting cover 10 and the handhold means 2, such that at the end of the threaded section, the pad protecting cover 10 can be released from the handhold means 2 either for separating the cover 10 from the pad 8, or to make the cover 10 slide relative to the handhold means 2 into the pad releasing position.

Still in another alternative embodiment the first retaining means 18 can be a bayonet coupling.

Still in another alternative embodiment the first retaining means 18 can be a predetermined breaking point allowing the separation and relative movement of the pad protecting cover 10 relative to the handhold means 2 into the pad releasing position.

The protecting cover 10 further comprises a plurality of first inlet through holes 12. The plurality of first inlet through holes 12 is open towards the collection pad 8 to provide a fluidical connection with the collection pad 8 when the pad protecting cover 10 is in the pad covering position, during the saliva sample collection procedure. Thanks to the first inlet through holes 12, when the collection device 1 is inserted in the user's oral cavity, the saliva can freely flow towards the collection pad 8 to wet it and collect as much saliva as possible.

In the Figures, it can be observed that preferably, the pad protecting cover 10 is a cylindrical sleeve, open at both ends, such that the remote end of the sleeve is indeed one of the through holes 12. In this preferred embodiment, the sleeve is dimensioned to allow the saliva collection pad 8 to protrude when the protecting cover 10 is withdrawn with a compressive effort on a free edge 134 of the cover 10, as shown in Figures 5 to 7.

Also to increase the quantity of saliva collected by the saliva collection device 1, in the invention it is provided that the device 1 further comprises a salivation enhancing layer 14 of a saliva properties-preserving composition. This salivation enhancing layer 14 is preferably a candy layer. As it is apparent from Figure 2, this salivation enhancing layer 14 covers at least partially the cover 10 in the longitudinal direction. Furthermore, to guarantee that as much saliva as possible is collected, the salivation enhancing layer has a plurality of second inlet through holes 16. These first and second inlet through holes 12, 16 match each other at least partially, but preferably completely to facilitate that the saliva reaches the saliva collection pad 8.

When the collection device 1 has a salivation enhancing layer 14, for better saliva collection, is desirable that the collection process with the saliva collection device 1 finishes once the salivation enhancing layer or the candy is fully solved in saliva. In other words, the user could start with the device of Figure 2 and finish the collection procedure with the appearance of Figure 3.

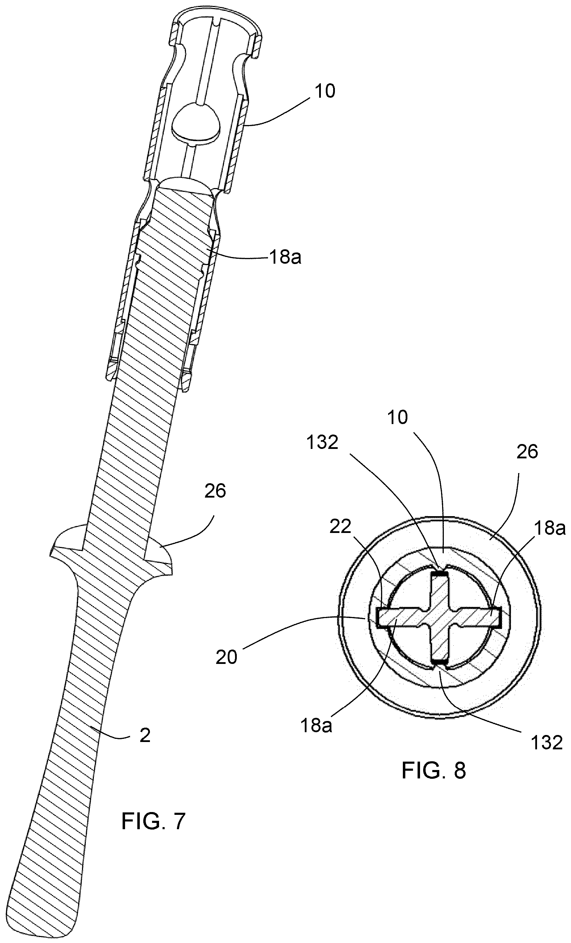

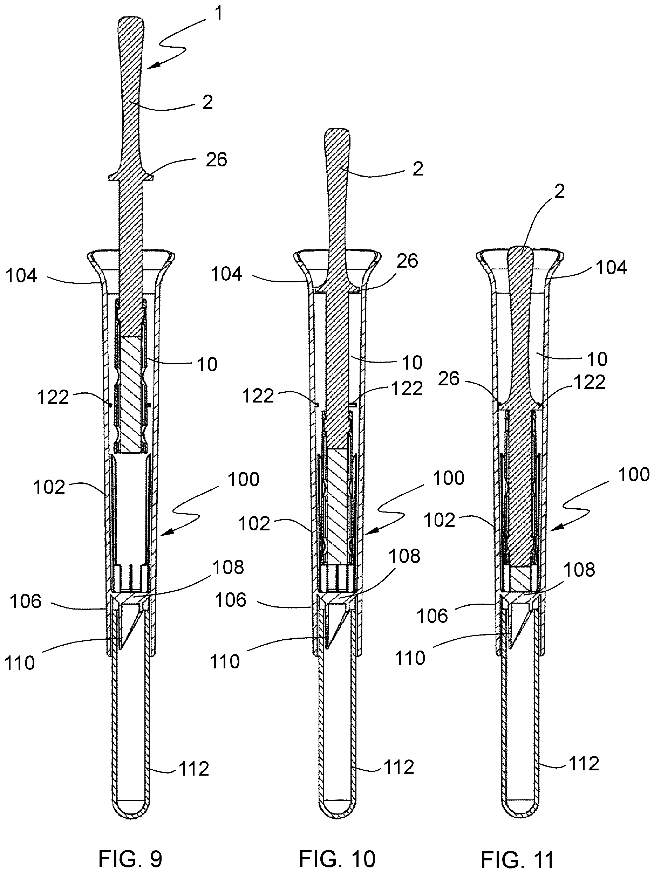

As it is apparent from Figure 8, the saliva collection device 1, also comprises first longitudinal guiding means 20 arranged between the pad protecting cover 10 and the handhold means 2 for guiding a longitudinal sliding movement of the pad protecting cover 10 and the handhold means 2 relative to each other, when the pad protecting cover 10 is moved from the pad 8 covering position into the pad releasing position, as shown in Figures 9 to 11. During this sliding movement, the pad protecting cover 10 and the handhold means 2 can freely slide relative to each other in a longitudinal sliding movement of the cover 10 in the longitudinal direction L from the first end 4 towards said second end 6.

The first longitudinal guiding means 20 of this embodiment comprises two guiding recesses 22 and corresponding guiding protrusions, which are in this case the protrusions 18b, thus fulfilling two functions in one. The guiding recess 22 and the guiding protrusion 18b cooperate mutually for guiding the longitudinal sliding movement when the pad protection cover 10 moves from a pad covering position to the pad releasing position. Also, for an even better improved guiding in the collection device 1, also secondary guiding means 132 are provided.

As explained above, the invention also seeks to facilitate the extraction once the sample has been collected with a saliva collection device 1 as the one explained above, reduces the risk of cross contamination and leakage of the sample and maximizes the saliva extraction from the saliva collected before with the saliva collection device.

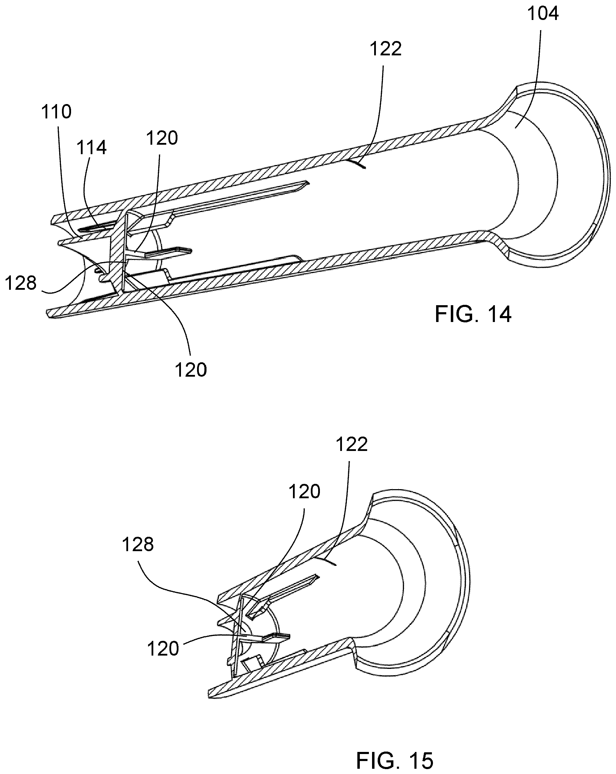

To solve this problem, the invention proposes a saliva extraction device 100 for extracting a saliva sample from a saliva collection device 1 according to the invention. Different features of the saliva extraction device 100 are shown in Figures 9 to 18.

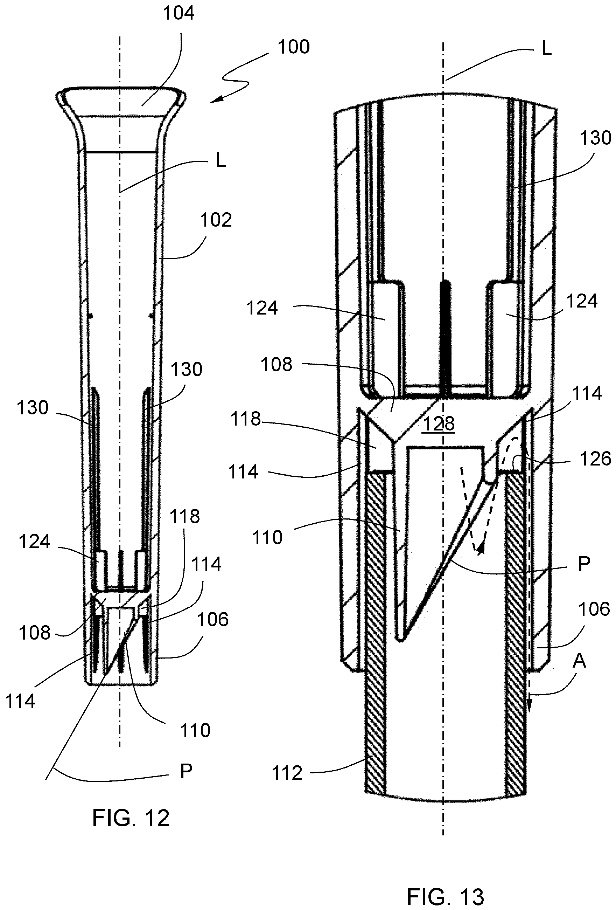

As it is apparent from Figure 12, the saliva extraction device 100 comprises a, preferably transparent saliva extraction cylinder 102 extending in a longitudinal direction L and comprising a third and a fourth ends 104, 106 and a compression wall 108 between the third and fourth ends 104, 106.

The compression wall 108 comprises a saliva outlet nozzle 110. The saliva outlet nozzle 110 protruding from the compression wall 108 towards the fourth end 106.

The saliva extraction cylinder 102 also comprises cover withdrawing means 124 arranged to the side of said wall 108 opposite to the saliva outlet nozzle 110. As it will explained below this cover withdrawing means 124 are provided for withdrawing the cover 10 of the saliva collection device 1 when a free edge 134 of the pad protecting cover 10 is abutting against the cover withdrawing means 124. This compression withdraws de cover 10 and the pad 8 can be fully compressed against the compression wall 108 for extracting a saliva sample contained in the pad 8 through the saliva outlet nozzle 110 into the saliva sample container 112.

The saliva extraction device 100 further comprises a, preferably transparent saliva sample container 112 fluidly connected to the saliva outlet nozzle 110 for receiving the saliva sample coming from the saliva collection device 1.

Finally, the saliva extraction device 100 also comprises securing means between the saliva extraction cylinder 102 and the saliva sample container 112 to removably secure the saliva sample container 112 to the saliva extraction cylinder 102, such as to receive the saliva sample flowing from said saliva outlet nozzle 110.

The saliva extraction device 100 makes the extraction especially easy because the extraction cylinder 102 is further configured for receiving the saliva collection device 1 through the third end 104 in a movable manner between a remote position in which the pad 8 is uncompressed, and a final extraction position in which the pad protecting cover 10 is withdrawn to a pad releasing position with a free edge 134 of the pad protecting cover 10 is abutting against the cover withdrawing means 124 and the pad 8 is fully compressed against the compression wall 108 for extracting a saliva sample contained in the pad 8 through the saliva outlet nozzle 110 into the saliva sample container 112.

Also preferably, to provide a smooth insertion, the saliva extraction cylinder 102 inner face tapers in the direction from said third end 4 towards said compression wall 108 and in this case comprises four longitudinal insertion ribs 130 tapering in the direction from the third end 104 towards said compression wall 108.

The extraction cylinder in this embodiment is 14 cm long, such that it can be hold very conveniently in one user's hand. Then when from the position of Figure 9 the user compresses the saliva collection device 1 against the cover withdrawing means 124 with a compression force with the thumb comprised between 20 N and 60 N, the position of Figure 11 is reached in a comfortable and secure way to avoid cross contamination of the saliva sample.

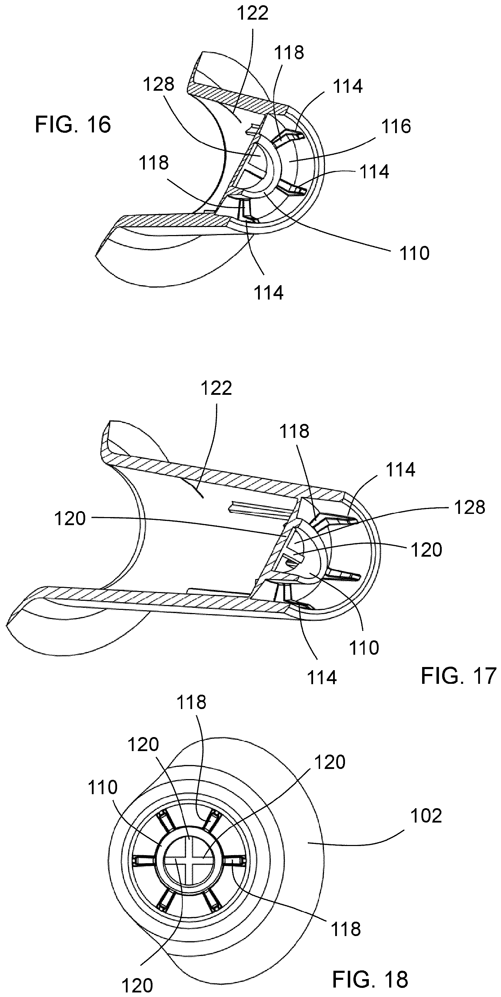

As shown in figures 16 to 18, in this embodiment, the securing means comprises six first longitudinal ribs 114 formed in the saliva extraction cylinder 102 to provide a press fit securing of the saliva sample container 112 onto the saliva extraction cylinder 102. The saliva sample container can be thus secured and removed very easily.

Additionally, and to avoid the bubble formation in the saliva sample between the saliva outlet nozzle 110 and the saliva sample container 112 at least one air outlet 116 is formed. In particular, this air outlet 116 is created because the compression wall 108 comprises six abutment protrusions 118 coincident with the longitudinal ribs 114 on the side of the outlet nozzle 110. When the saliva sample container 112 is removably secured to the saliva extraction cylinder 102 a front edge 126 of the saliva sample container 112 abuts against said first abutment protrusion 118. Figure 13, by means of the curved arrow A shows how this air outlet 116 works as a labyrinth to avoid saliva leakages but avoiding the bubble formation and reducing the amount of already existing bubbles in the sample.

Furthermore, to reach a high volume of draining of the saliva collection pad 8, the compression wall 108 of this embodiment comprises four extraction ribs 120 on the side of said compression wall 108 opposite to the outlet nozzle 110 created in the inlet hole of the outlet nozzle. Therefore, in the compressed position, in which the pad 8 is compressed against the compression wall 108 for extracting a saliva sample, the pad 8 is compressed against the four extraction ribs 120. Furthermore, these four extraction ribs 120 create a draining chamber 128 that provides for the saliva sample accumulation and fluidification to flow downwards and avoids that the saliva collection pad 8 draws the sample back, thus reducing the amount of collected saliva.

Also in this embodiment, to accelerate the flow between the draining chamber 128 and the saliva sample container 112, it is provided that on the side of said compression wall 108 opposite to the outlet nozzle 110, the compression wall 108 is shaped as a funnel and said funnel flows into said saliva outlet nozzle 110, as shown in Figure 13.

To further improve the saliva extraction process, the saliva outlet nozzle 110 is shaped as a tubular duct extending in the longitudinal direction L and the end of the tubular duct remote from the compression wall 108 is finished in a plane P diagonally oriented relative to said longitudinal direction L, as it is apparent from Figures 12 and 13.

Finally, to help the user to perform a correct saliva extraction process, the handhold means 12 comprises a flange 26 and the saliva extraction cylinder 102 comprises at two final stroke protrusions 122 on the inner surface of the saliva extraction cylinder 102, the flange 26 and the final stroke protrusion 122 cooperate mutually to produce a tactile feedback when the saliva collection device 1 has reached said final extraction position.

Especially preferably the pad protecting cover 10 is so long that in the final extraction position in which said pad protecting cover 10 is withdrawn to said pad releasing position, the cover 10 simultaneously abuts against said flange 26 and the cover withdrawing means 124. This provides also a reliable feeling to the user that the extraction is performed correctly.

Below a further embodiment of the saliva collection device and the saliva extraction device are shown. These embodiments share many features in common with the embodiments already described. Therefore, now only the distinguishing features will be described, while for the features in common, reference is made to the previous passages.

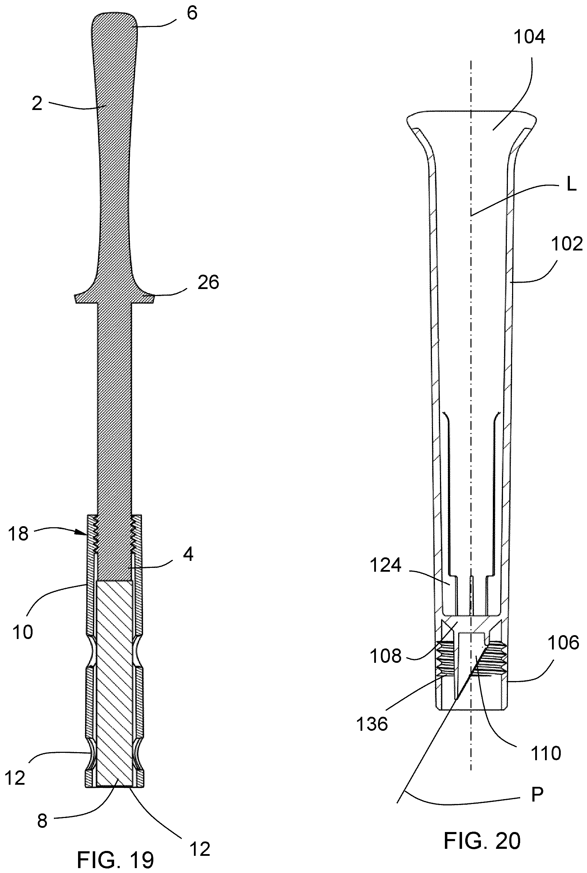

In an alternative embodiment of the saliva collection device, shown in Figure 19 and seeking for a more sealing retention, the first retaining means 18 comprises matching threads between the pad protecting cover 10 and said handhold means 2, for releasably securing said pad protecting cover and said handhold means 2, relative to each other in said pad covering position.

In this case, rather than a compression force, to move the pad protecting cover 10 from the pad covering position to the pad release position, a torque must be applied to turn one part relative to the other until the end of the threaded joint.

Finally, in relation to Figure 20 in this case, the securing means comprises matching threads 136 between said extraction cylinder 102 and said saliva sample container 112 (not shown in this Figure) to provide a threaded connection between both parts.

## Claims

1. A saliva collection device (1) for collecting a saliva sample of a user comprising:
[a] handhold means (2) with a first and a second ends (4, 6), said handhold means (2) extending in a longitudinal direction (L), and
[b] an absorbent saliva collection pad (8), said collection pad (8) being secured to said first end (4) of said handhold means (2) to extend in said longitudinal direction (L), **characterized in that** it further comprises
[c] a pad protecting cover (10), said protecting cover (10)
[i] being releasably secured to said handhold means (2) in a pad covering position, in which said pad (8) is protected from deformation during a saliva sample collection procedure, and
[ii] being movable relative to said handhold means (2), out of said pad covering position, to a pad releasing position, to release said collection pad (8) after said saliva sample collection procedure has been finished, to extract said saliva sample, and
[d] said protecting cover (10) further comprising a plurality of first inlet through holes (12), said plurality of first inlet through holes (12) being open towards said collection pad (8) to provide a fluidical connection with said collection pad (8) when said pad protecting cover is in said pad covering position during said saliva sample collection procedure.

2. The saliva collection device (1) of claim 1, **characterized in that** it further comprises a salivation enhancing layer (14), said salivation enhancing layer (14) being made of a saliva properties-preserving composition and covering at least partially said cover (10) in the longitudinal direction and comprising a plurality of second inlet through holes (16), said first and second inlet through holes (12, 16) matching each other at least partially.

3. The saliva collection device (1) of claim 2, **characterized in that** said salivation enhancing layer (14) is preferably a candy layer.

4. The saliva collection device (1) of any one of claims 1 to 3, **characterized in that** it comprises first retaining means (18) arranged between said pad protecting cover (10) and said handhold means (2), for releasably securing said pad protecting cover (10) and said handhold means (2) relative to each other in said pad (8) covering position.

5. The saliva collection device (1) of claim 4, **characterized in that** said first retaining means (18) comprises at least one retaining hole (18a) and at least matching first retaining protrusion (18b), functionally arranged in said pad protecting cover (10) and said handhold means (2), such that in said pad (8) covering position, said first retaining protrusion (18b) is inserted into said at least one retaining hole (18a).

6. The saliva collection device (1) of any one of claims 1 to 5, **characterized in that** it comprises first longitudinal guiding means (20) arranged between said pad protecting cover (10) and said handhold means (2), for guiding a longitudinal sliding movement of said pad protecting cover (10) and said handhold means (2) relative to each other, when said pad protecting cover (10) is moved from said pad (8) covering position into said pad releasing position during which said pad protecting cover (10) and said handhold means (2) can freely slide relative to each other in a longitudinal sliding movement in said longitudinal direction (L) from said first end (4) towards said second end (6).

7. The saliva extraction device of any one of claims 1 to 5, **characterized in that** said first longitudinal guiding means (20) comprises at least one guiding recess (22) and a guiding protrusion and **in that** said guiding recess (22) and said guiding protrusion cooperate mutually for guiding said longitudinal sliding movement.

8. A saliva extraction device (100) for extracting a saliva sample from a saliva collection device (1) **according to any of the claims 1 to 7, characterized in that** said saliva extraction device (100) further comprises
[a] a saliva extraction cylinder (102) extending in a longitudinal direction (L) and comprising a third and a fourth ends (104, 106) and a compression wall (108) between said third and fourth ends (104, 106), said wall (108) comprising a saliva outlet nozzle (110) protruding from said compression wall (108) towards said fourth end (106) and cover withdrawing means (124) arranged to the side of said wall (108) opposite to said saliva outlet nozzle (110) for withdrawing said cover (10) of said saliva collection device (1),
[b] a saliva sample container (112) fluidly connected to said saliva outlet nozzle (110) for receiving said saliva sample, and
[c] securing means between said saliva extraction cylinder and said saliva sample container to removably secure said saliva sample container (112) to said saliva extraction cylinder (102), such as to receive said saliva sample flowing from said saliva outlet nozzle (110),
[d] said extraction cylinder (102) being further configured for receiving said saliva collection device (1) through said third end (104) in a movable manner between
[i] a remote position in which said pad (8) is uncompressed, and
[ii] a final extraction position in which said pad protecting cover (10) is withdrawn to a pad releasing position with a free edge of said pad protecting cover (10) abutting against said cover withdrawing means (124) and said pad (8) is fully compressed against said compression wall (108) for extracting a saliva sample contained in said pad (8) through said saliva outlet nozzle (110) into said saliva sample container (112).

9. The saliva extraction device of claim 8, **characterized in that** said securing means comprises at least three first longitudinal ribs (114) formed in said saliva extraction cylinder (102) to provide a press fit securing of said saliva sample container (112) onto said saliva extraction cylinder (102).

10. The saliva extraction device of claim 8 or 9, **characterized in that** between said saliva outlet nozzle 110 and said saliva sample container (112) at least one air outlet (116) is formed.

11. The saliva extraction device of claim 10, **characterized in that** said compression wall (108) comprises at least a first abutment protrusion (118) on the side of said outlet nozzle (110) and when said saliva sample container (112) is removably secured to said saliva extraction cylinder (102) a front edge (126) of said saliva sample container (112) abuts against said first abutment protrusion (118).

12. The saliva extraction device of any one of claims 8 to 11, **characterized in that** said compression wall (108) comprises at least three extraction ribs (120) on the side of said compression wall (108) opposite to said outlet nozzle (110) protruding from said compression wall (108), such that in said compressed position, in which said pad (8) is compressed against said compression wall (108) for extracting a saliva sample, said pad (8) is compressed against said at least three extraction ribs (120).

13. The saliva extraction device of any one of claims 8 to 12, **characterized in that** on the side of said compression wall (108) opposite to said outlet nozzle (110), said compression wall (108) is shaped as a funnel, such that in use said collected saliva can flow into said saliva outlet nozzle (110).

14. The saliva extraction device of any claims 8 to 13, **characterized in that** said saliva outlet nozzle (110) is shaped as a tubular duct extending in the longitudinal direction (L) and **in that** the end of said tubular duct remote from said compression wall (108) is finished in a plane (P) diagonally oriented relative to said longitudinal direction (L).

15. The saliva extraction device of any claims 8 to 14, **characterized in that** said handhold means (12) comprises a flange (26) and said saliva extraction cylinder (102) comprises at least one final stroke protrusion (122) on the inner surface of said saliva extraction cylinder (102), said flange (26) and said final stroke protrusion (122) cooperate mutually to produce a tactile feedback when said saliva collection device (1) has reached said final extraction position.
